# EUROPÄISCHE PATENTANMELDUNG

(11) **EP 1 270 534 A1**
(43) Veröffentlichungstag der Anmeldung: **02.01.2003**
(21) Anmeldenummer: 01810613.8
(22) Anmeldetag: 22.06.2001
(51) Int. Cl.: C04B 41/87, A61F 9/013

(54) **Skalpellklinge für Katarakt-Operationen**

(71) Anmelder: SIS AG, Surgical Instruments Systems, 2555 Brügg (CH)
(72) Erfinder: Die Erfindernennung liegt noch nicht vor
(74) Vertreter: Clerc, Natalia

(57) **Zusammenfassung**

In einem Verfahren zur Herstellung einer Katarakt-Skalpellklinge aus Keramik wird die Skalpellklinge als poröse Rohform in einer Edelgas-Atmosphäre heiss-isostatisch gepresst, um Poren der Rohform zu verschweissen. Darauf wird die verschweisste Poren aufweisende Rohform geläppt, die geläppte Rohform zu einer Klinge vorgeschliffen, die vorgeschliffene Klinge poliert, die polierte Klinge gereinigt und die gereinigte Klinge mit amorphem Kohlenstoff beschichtet.

## Beschreibung

### Technisches Gebiet

Die Erfindung betrifft ein Verfahren zur Herstellung einer Ophtalmologie-Skalpellklinge und eine Ophtalmologie-Skalpellklinge gemäss Oberbegriff des Patentanspruches 1 beziehungsweise 11. Die Klinge eignet sich vorzugsweise für die Durchführung von Katarakt-Operationen.

### Stand der Technik

Als Katarakt oder Grauer Star bezeichnet man eine Eintrübung einer ursprünglich klaren Augenlinse. Dieser Katarakt lässt sich operieren, indem die getrübte Augenlinse entfernt und anderweitig für eine optische Korrektur gesorgt wird. Zur Entfernung der Augenlinse wird in einem mikrochirurgischen Eingriff mit einem sehr feinen Skalpell ein Kanal eröffnet. Über diese Öffnung wird dann die natürliche Linse mittels Ultraschall zertrümmert. Die Öffnung wird mit einem weiteren mikrochirurgischen Schnitt erweitert, um eine Ersatzlinse einzusetzen.

Üblicherweise wird diese Klinge aus Stahl oder Diamant hergestellt. Stahlklingen haben jedoch den Nachteil, dass ihre Oberfläche relativ rau ist. Die raue Oberfläche führt dazu, dass sie sich nicht genügend reinigen lässt und dass ein gewisser Druck notwendig ist, um einen Schnitt auszuführen. Zudem lösen sich bei Operationen zum Teil Metallpartikel von der Klinge, welche im Auge verbleiben und zu Entzündungen führen können. Diamantklingen benötigen einen kleineren Druck beim Schneiden und weisen eine Vielzahl bekannter Vorteile auf. Nachteilig ist hingegen, dass ihre Herstellung relativ aufwendig ist und die Klinge somit relativ teuer ist.

Ferner sind aus dem Stand der Technik Keramikklingen bekannt. So offenbart DE-A-196'52'098 ein Skalpell, welches einstückig aus einer Keramik, insbesondere aus Zirkondioxid oder aus einer Mischung aus Zirkondioxid und Aluminiumoxid, hergestellt ist. Dabei wird das Skalpell in einem Spritzgussverfahren einstückig aus einem keramischen Werkstück hergestellt, vorhandene Kunststoffteile entbindert und das Werkstück dann gesintert. Diese Klingen haben mehrere Vorteile. Sie sind elektrisch nicht leitend, weisen eine hohe Festigkeit auf, besitzen eine ausreichende Rückstellkraft und sind antiallergen. Zudem lassen sie sich wie die Diamantklingen nachschleifen. Derartige Klingen genügten bis jetzt jedoch nicht den erhöhten Anforderungen, welche in der Ophtalmologie, insbesondere bei Katarakt-Operationen, an Skalpellklingen gestellt werden.

### Darstellung der Erfindung

Es ist deshalb eine Aufgabe der Erfindung, ein Verfahren zur Herstellung einer Ophtalmologie-Skalpellklinge und eine Ophtalmologie-Skalpellklinge zu schaffen, welche in der Ophtalmologie, insbesondere bei Katarakt-Operationen, einsetzbar sind.

Diese Aufgabe löst eine Vorrichtung und ein Verfahren mit den Merkmalen des Patentanspruches 1 beziehungsweise 11.

Erfindungsgemäss wird mindestens die Oberfläche einer gesinterten keramischen Rohform mittels heiss-isostatischer Pressung verdichtet, wobei Poren der Keramik verschweisst werden, diese Rohform geläppt, vorgeschliffen und eine entstandene Klinge poliert, gereinigt und mit amorphem Kohlenstoff beschichtet wird.

Diese Klinge ist sehr hart, jedoch nicht spröde. Die Bruchgefahr ist aufgrund der hohen Biegebruchfestigkeit sehr gering. Zudem können sich bei Operationen keine Partikel lösen.

Diese Keramikklinge eignet sich insbesondere zum Schneiden der Hornhaut oder Sklera. Der Reibungskoeffizient zwischen der erfindungsgemäss behandelten Keramikklinge und der Hornhaut oder Sklera eignet sich auch für ein Eindringen in tiefere Strukturen des Gewebes, ohne dass viel Kraft aufgewendet werden muss. Dies verbessert die Schnittleistung im Vergleich zu Stahl-, aber auch zu Diamantklingen.

Die geläppte Oberfläche lässt eine Reinigung zu, welchen den in der Ophtalmologie gestellten Anforderungen genügt. Nach der Herstellung sind keine abrasiven Rückstände wie bei der Stahlklinge feststellbar. Zudem lässt sich die erfindungsgemässe Klinge auch nach den chirurgischen Eingriffen bedeutend besser von Geweberesten und Blut reinigen.

Weitere vorteilhafte Varianten des Verfahrens und vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine perspektivische Darstellung einer Skalpellklinge;
- Figur 2: eine Frontansicht der Klinge gemäss Figur 1 und
- Figur 3: eine Seitenansicht der Klinge gemäss Figur 1.

### Wege zur Ausführung der Erfindung

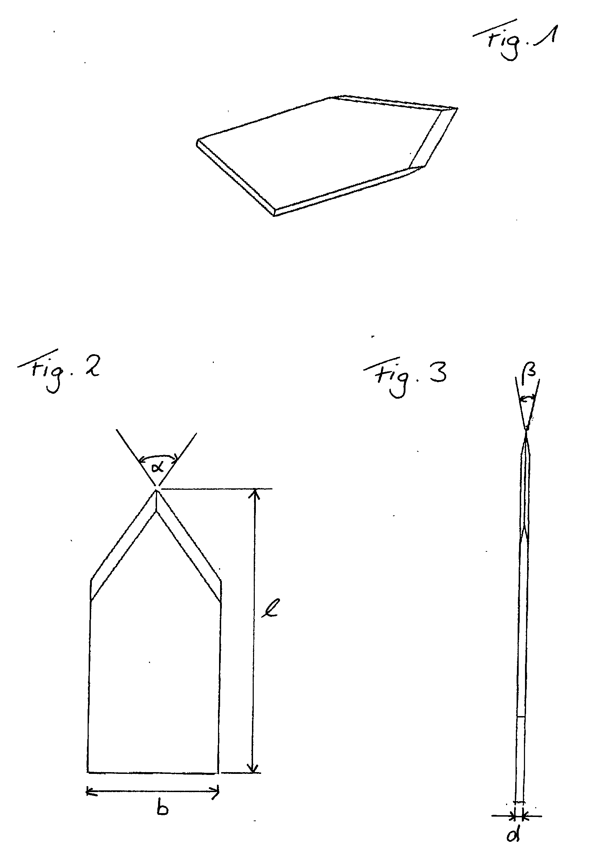

In den Figuren 1 bis 3 ist eine Skalpellklinge dargestellt, wie sie typischerweise für Katarakt-Operationen eingesetzt wird. Andere Formen sind jedoch auch möglich. Diese Klinge ist lös- oder unlösbar in einem hier nicht dargestellten Skalpellgriff gehalten. Typische Abmessungen für derartige Skalpellklingen betragen für ihre Breite b 3.0 - 3.2 mm, für ihre Länge 1 7 mm, für ihre Dicke d 0.18 - 0.2 mm, für ihren ersten Schneidwinkel α 60°-70° und für ihren zweiten Schneidwinkel β 36°. Je nach Anwendungsbereich sind andere Masse möglich. Die obengenannten Schnittwinkel sind jedoch für Katarakt-Operationen besonders gut geeignet.

Zur Herstellung einer derartigen Skalpellklinge wird im erfindungsgemässen Verfahren als Ausgangsmaterial eine gesinterte Keramik eingesetzt. Vorzugsweise weist diese eine Rohform auf, welche unter Berücksichtigung der nachfolgend beschriebenen Verfahrensschritte bereits im wesentlichen die gewünschte äussere Endform der Klinge aufweist. Als Keramik wird ein Material mit möglichst engen Kugelpackung eingesetzt. Vorzugsweise wird als Ausgangsmaterial Zirkondioxid verwendet. Die unten stehenden, im Verfahren angegebenen Parameter beziehen sich, wenn auch nicht ausschliesslich, auf eine Klinge aus Zirkondioxid, welche die obengenannten Endabmessungen aufweist.

In einem ersten Schritt wird die poröse Rohform in einer Edelgas-Atmosphäre in einem Autoklaven heiss-isostatisch gepresst. Dank dieser heiss-isostatischen Pressung, auch Hipen genannt, wird die Kugelpackung noch mehr verdichtet. Die Poren der Rohform werden verschweisst. Durch richtige Kombination von Druck, Temperatur und Einwirkzeit werden die Gefügeeigenschaften der Rohform und somit der resultierenden Skalpellklinge positiv verändert.

In einer bevorzugten Variante erfolgt die heiss-isostatische Pressung mit Argon unter einem Gasdruck von vorzugsweise 1350 bar. Die Temperatur im Autoklaven beträgt vorzugsweise 1960°C. Die Aufheizphase beträgt 2.5 Stunden und die Haltephase 1.6 Stunden. Nach der heiss-isostatischen Pressung wird die Rohform innerhalb von 4.5 Stunden auf Umgebungstemperatur abgekühlt.

In einem nächsten Schritt wird die Rohform geläppt. Vorzugsweise wird hierfür ein monokristallines Diamantpulver mit einer Körnung von 0.5 - 0.75 µm verwendet.

Die geläppte Rohform wird nun zu einer Klinge vorgeschliffen. Gute Resultate wurden mit einer Schleifscheibe erzielt, welche aus Bronze besteht, eine Körnung von D7, einen Scheibendurchmesser von 150 mm, eine Belagsbreite von 15 mm und eine Belagstiefe von 4 mm aufweist.

Nachfolgend wird die vorgeschliffene Klinge poliert, wobei vorzugsweise mit einem monokristallinen Diamantpulver mit einer Körnung von maximal 0.1 µm poliert wird.

Nun wird die polierte Klinge in mehreren Schritten gereinigt. In einem ersten Schritt wird sie in einem ersten Ultraschallbad mit N-metyl-pyrolidin entfettet, in einem zweiten Schritt mit destilliertem Wasser gespült, in einem dritten Schritt in einem zweiten Ultraschallbad mit Isopropyl gereinigt, in einem vierten Schritt mit destilliertem Wasser gespült und in einem fünften Schritt unter Verwendung von Sauerstoff plasmagereinigt.

Schliesslich wird die gereinigte Klinge mit amorphem Kohlenstoff beschichtet. Vorzugsweise weist diese Beschichtung eine Dicke von vorzugsweise von 0.1 µm, auf.

Die mit dem oben beschriebenen Verfahren hergestellte Skalpellklinge erfüllt alle Anforderungen der Ophtalmologie, insbesondere der Katarakt-Chirurgie, und weist die bei Stahl- und Diamantklingen bekannten Nachteile nicht auf.

## Patentansprüche

1. Verfahren zur Herstellung einer Katarakt-Skalpellklinge aus Keramik, wobei, die Skalpellklinge als poröse Rohform in einer Edelgas-Atmosphäre heiss-isostatisch gepresst wird, um Poren der Rohform zu verschweissen, wobei die verschweisste Poren aufweisende Rohform geläppt wird, wobei die geläppte Rohform zu einer Klinge vorgeschliffen wird, wobei die vorgeschliffene Klinge poliert wird, wobei die polierte Klinge gereinigt wird und wobei die gereinigte Klinge mit amorphem Kohlenstoff beschichtet wird.

2. Verfahren nach Anspruch 1, wobei die heiss-isostatische Pressung bei einem Druck von 1350 bar erfolgt.

3. Verfahren nach Anspruch 1, wobei als Edelgas Argon verwendet wird.

4. Verfahren nach Anspruch 1, wobei die heiss-isostatische Pressung bei einer Temperatur von 1960°C erfolgt.

5. Verfahren nach Anspruch 1, wobei die heiss-isostatische Pressung in einer Aufheizphase während 2.5 Stunden und in einer Haltephase während 1.6 Stunden erfolgt.

6. Verfahren nach Anspruch 1, wobei die Rohform nach der heiss-isostatischen Pressung innerhalb von 4.5 Stunden auf Umgebungstemperatur abgekühlt wird.

7. Verfahren nach Anspruch 1, wobei mit einem monokristallinem Diamantpulver mit einer Körnung von 0.5 - 0.75 µm geläppt wird.

8. Verfahren nach Anspruch 1, wobei mit einem monokristallinem Diamantpulver mit einer Körnung von maximal 0.1 µm poliert wird.

9. Verfahren nach Anspruch 1, wobei die polierte Klinge in mehreren Schritten gereinigt wird, wobei sie in einem ersten Schritt in einem ersten Ultraschallbad mit N-metyl-pyrolidin entfettet wird, in einem zweiten Schritt mit destilliertem Wasser gespült wird, in einem dritten Schritt in einem zweiten Ultraschallbad mit Isopropyl gereinigt wird, in einem vierten Schritt mit destilliertem Wasser gespült wird und in einem fünften Schritt unter Verwendung von Sauerstoff plasmagereinigt wird.

10. Verfahren nach Anspruch 1, wobei die amorphe Kohlenstoffbeschichtung in einer Dicke von 0.1 µm, aufgebracht wird.

11. Katarakt-Skalpellklinge aus Keramik, **dadurch gekennzeichnet, dass** ihre Poren verschweisst sind und dass sie eine Beschichtung aus amorphem Kohlenstoff aufweist.
